# EUROPÄISCHE PATENTANMELDUNG

(11) **EP 3 435 261 A1**
(43) Veröffentlichungstag der Anmeldung: **30.01.2019**
(21) Anmeldenummer: 18170360.4
(22) Anmeldetag: 02.05.2018
(51) Int. Cl.: G06F 19/00, A61B 10/00, G16H 40/63, G16H 50/50, G16H 50/20, G16H 50/70

(54) **VORHERSAGE DES BLUTUNGSVERHALTENS VON FRAUEN NACH EINSETZEN EINES INTRAUTERINEN WIRKSTOFFFREISETZUNGSSYSTEMS**

(30) Priorität: 28.07.2017 EP 17183705
(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung befasst sich mit der Vorhersage des Regelblutungsverhaltens bei Frauen, denen ein intrauterines Wirkstofffreisetzungssystems eingesetzt worden ist. Gegenstände der vorliegenden Erfindung sind ein System, ein Verfahren und ein Computerprogrammprodukt zur Vorhersage des Regelblutungsverhaltens.

## Beschreibung

Die vorliegende Erfindung befasst sich mit der Vorhersage des Regelblutungsverhaltens bei Frauen, denen ein intrauterines Wirkstofffreisetzungssystems eingesetzt worden ist. Gegenstände der vorliegenden Erfindung sind ein System, ein Verfahren und ein Computerprogrammprodukt zur Vorhersage des Regelblutungsverhaltens.

Ein intrauterines Wirkstofffreisetzungssystem ist ein Intrauterinpessar, das einen oder mehrere Wirkstoffe umfasst und in die Gebärmutter einer Frau eingesetzt wird. Dort gibt es den einen Wirkstoff oder die mehreren Wirkstoffe langsam über einen längeren Zeitraum ab. Ein Beispiel für ein intrauterines Wirkstofffreisetzungssystem ist eine Hormonspirale, die langsam und gleichmäßig ein Gestagen in der Gebärmutter abgibt, z.B. um eine Schwangerschaft zu verhüten und/oder zur Therapie von Idiopathischer Menorraghie oder Dysmenorrhoe.

Im natürlichen Verlauf des Zyklus baut sich die Gebärmutterschleimhaut auf und wird beim Ausbleiben einer Schwangerschaft abgestoßen und über den Zervikalkanal durch die Vagina ausgeschieden. Dies wird als Menstruationsblutung wahrgenommen. Je nachdem wie hoch die Schleimhaut aufgebaut wird, kann die Regelblutung unterschiedlich stark sein.

Bei Verwendung eines intrauterinen Wirkstofffreisetzungssystems wird der Aufbau der Gebärmutterschleimhaut durch den freigesetzten Wirkstoff beeinflusst. Zum Beispiel baut sich bei Verwendung einer Gestagen-enthaltenden Hormonspirale aufgrund der Gestagenwirkung die Gebärmutterschleimhaut nur schwach auf. Die Regelblutungen werden leichter, weniger schmerzhaft und können auch ganz ausbleiben (siehe z.B. D. Socolov et al., The European Journal of Contraception and Reproductive Health Care, December 2011; 16: 480-487).

Intrauterine Wirkstofffreisetzungssysteme können demnach zu einem veränderten Blutungsverhalten führen. Insbesondere kann es in der Eingewöhnungsphase zu unvorhergesehenen Blutungen kommen, was für eine Frau, die vorher regelmäßige Regelblutungen hatte, eine negative Erfahrung darstellt.

Manche Frauen sind daher bei der ersten Verwendung eines intrauterinen Wirkstofffreisetzungssystems verunsichert und/oder unzufrieden. Manche Frauen lassen sich das eingesetzte intrauterine Wirkstofffreisetzungssystem frühzeitig wieder entnehmen - gegebenenfalls hätte sich das Blutungsverhalten bei diesen Frauen aber noch auf ein verlässliches Muster eingestellt. Bei anderen Frauen wiederum kann es sein, dass dauerhaft Beschwerden auftreten, so dass die Entnahme des intrauterinen Wirkstofffreisetzungssystems sinnvoll sein kann.

Es wäre demnach vorteilhaft, wenn man das Blutungsverhalten einer Frau, die ein intrauterines Wirkstofffreisetzungssystem verwendet, vorhersagen könnte.

Im Stand der Technik ist keine solche Lösung beschrieben.

Es gibt Lösungen zur Vorhersage des Menstruationszyklus (US2016/0196383A1, US2016/0100826A1), insbesondere zur Ermittlung der fruchtbaren Tage (US2010/0191696A1, US5,836,890), diese sind jedoch für Frauen, die ein intrauterines Wirkstofffreisetzungssystem tragen, nicht anwendbar. E.T. de Jonge *et al.* beschäftigten sich im Rahmen einer Studie mit der Identifizierung von Prädiktoren für Oligomenorrhoe bei Frauen, die ein intrauterinen Wirkstofffreisetzungssystems tragen (Contraception 76 (2007) 91-95). Der dort gewählte Ansatz ist jedoch auf eine Vorhersage des allgemeinen Blutungsverhaltens nicht übertragbar. Ziel der Studie war, Faktoren vor Einsetzen des intrauterinen Wirkstofffreisetzungssystems zu identifizieren, die mit einer Oligomenorrhoe 12 Monate nach Einsetzen verbunden sind. Zum einen ist eine Vorhersage für einen Zeitpunkt, der 12 Monate nach Einsetzen des intrauterinen Wirkstofffreisetzungssystems liegt, zeitlich vergleichsweise spät: es wäre für viele Frauen von Vorteil, bereits zu einem früheren Zeitpunkt eine Aussage zum zukünftigen Blutungsverhalten zu erhalten. Zum anderen, und das bestätigen eigene Untersuchungen, erweist sich eine Vorhersage des Blutungsverhaltens allein auf Basis von Prädiktoren, die vor Einsetzen des intrauterinen Wirkstofffreisetzungssystems erhoben werden, als nicht erfolgreich.

In den eigenen Arbeiten, die der vorliegenden Erfindung zu Grunde liegen, wurde ein anderer Ansatz verfolgt, der sich als erfolgreich herausstellte: es wurden in den Daten einer Studie zum Blutungsverhalten von Frauen, denen ein intrauterines Wirkstofffreisetzungssystems eingesetzt worden ist, für einen Zeitraum, der 61-180 Tage nach dem Einsetzen des intrauterines Wirkstofffreisetzungssystems liegt, Klassen mit jeweils ähnlichem Blutungsverhalten identifiziert und in einem weiteren Schritt geprüft, ob und wie sich diese Klassen aus Daten, die vor dem genannten Zeitraum erhoben worden sind, vorhersagen lassen. Dadurch konnte ein Vorhersagemodell entwickelt werden, das das Blutungsverhalten von Frauen auf Basis von Prädiktorenwerten, die in einem Beobachtungszeitraum erfasst werden, für einen Vorhersagezeitraum, der zeitlich an den Beobachtungszeitraum anschließt, vorhersagt.

Ferner wurde eine Lösung gefunden, wie sich die Regelmäßigkeit von vorhergesagten Blutungen definieren und ebenfalls vorhersagen lässt.

Ein erster Gegenstand der vorliegenden Erfindung ist ein System zur Vorhersage des Blutungsverhaltens einer Frau, der ein intrauterines Wirkstofffreisetzungssystem eingesetzt worden ist, umfassend
- eine Erfassungseinheit zur Erfassung von Prädiktorenwerten, wobei die Erfassungseinheit so konfiguriert ist, dass sie Informationen zum Blutungsverhalten der Frau in einer Beobachtungszeitspanne erfasst, wobei die Beobachtungszeitspanne mit dem Einsetzen des intrauterinen Wirkstofffreisetzungssystems beginnt und mindestens 60 Tage dauert,
- eine Vorhersageeinheit, die so konfiguriert ist, dass sie das zukünftige Blutungsverhalten der Frau auf Basis der Prädiktorenwerte ermittelt, wobei der Vorhersageeinheit ein Klassifikationsmodell zugrunde liegt, dass die Frau einer von mehreren Blutungsverhaltensklassen zuordnet, wobei sich die Blutungsverhaltensklassen hinsichtlich zu erwartender Blutungsintensitäten in einer Vorhersagezeitspanne unterscheiden, wobei die Vorhersagezeitspanne zeitlich an die Beobachtungszeitspanne anschließt,
- eine Ausgabeeinheit, die so konfiguriert ist, dass sie das ermittelte zukünftige Blutungsverhalten gegenüber der Frau und/oder einer anderen Person, insbesondere gegenüber einem Arzt, anzeigt.

Ein weiterer Gegenstand der vorliegenden Erfindung ist ein Verfahren zur Vorhersage des Blutungsverhaltens einer Frau, der ein intrauterines Wirkstofffreisetzungssystem eingesetzt worden ist, umfassend die Schritte
- Erfassen des Blutungsverhaltens der Frau in einer Beobachtungszeitspanne, wobei die Beobachtungszeitspanne mit dem Einsetzen des intrauterinen Wirkstofffreisetzungssystems beginnt und mindestens 60 Tage dauert, und optionales Erfassen weiterer Prädiktorenwerte,
- Ermitteln des zukünftigen Blutungsverhalten der Frau auf Basis der Prädiktorenwerte mit Hilfe eines Klassifikationsmodells, wobei das Klassifikationsmodell die Frau einer von mehreren Blutungsverhaltensklassen zuordnet, wobei sich die Blutungsverhaltensklassen hinsichtlich zu erwartender Blutungsintensitäten in einer Vorhersagezeitspanne unterscheiden, wobei die Vorhersagezeitspanne zeitlich an die Beobachtungszeitspanne anschließt,
- Ausgeben des ermittelten zukünftigen Blutungsverhaltens an die Frau und/oder an eine andere Person, insbesondere an einen Arzt.

Ein weiterer Gegenstand der vorliegenden Erfindung ist ein Computerprogrammprodukt umfassend einen Datenträger, auf dem ein Computerprogramm gespeichert ist, das in den Arbeitsspeicher eines oder mehrerer Computersysteme geladen werden kann und dort das Computersystem/die Computersysteme dazu veranlasst, folgende Schritte ausführen:
- Erfassen des Blutungsverhaltens einer Frau, der ein intrauterines Wirkstofffreisetzungssystem eingesetzt worden ist, in einer Beobachtungszeitspanne, wobei die Beobachtungszeitspanne mit dem Einsetzen des intrauterinen Wirkstofffreisetzungssystems beginnt und mindestens 60 Tage dauert, und optionales Erfassen weiterer Prädiktorenwerte,
- Ermitteln des zukünftigen Blutungsverhalten der Frau auf Basis der Prädiktorenwerte mit Hilfe eines Klassifikationsmodells, wobei das Klassifikationsmodell die Frau einer von mehreren Blutungsverhaltensklassen zuordnet, wobei sich die Blutungsverhaltensklassen hinsichtlich zu erwartender Blutungsintensitäten in einer Vorhersagezeitspanne unterscheiden, wobei die Vorhersagezeitspanne zeitlich an die Beobachtungszeitspanne anschließt,
- Ausgeben des ermittelten zukünftigen Blutungsverhaltens an die Frau und/oder an eine andere Person, insbesondere an einen Arzt.

Die Erfindung wird nachstehend näher erläutert, ohne zwischen den Erfindungsgegenständen (Verfahren, System, Computerprogrammprodukt) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt) sie erfolgen.

Die vorliegende Erfindung erlaubt eine Vorhersage des zukünftigen Blutungsverhaltens einer Frau, die ein intrauterines Wirkstofffreisetzungssystem trägt/verwendet.

Ein "intrauterines Wirkstofffreisetzungssystem" ist ein Intrauterinpessar, das in die Gebärmutter einer Frau eingesetzt wird und dort einen oder mehrere Wirkstoffe abgibt.

Ein "Wirkstoff' ist eine Substanz oder ein Substanzgemisch, die/das in einem Organismus eine spezifische Wirkung hat und eine spezifische Reaktion hervorruft. Insbesondere handelt es sich bei einem Wirkstoff im Sinne der vorliegenden Erfindung um eine Substanz oder ein Substanzgemisch, die/das eine Wirkung auf das Endometrium einer Frau ausübt.

Ein Beispiel für einen Wirkstoff ist ein Hormon, z.B. ein Gestagen.

"Gestagene" sind Steroide, die als Grundgerüst Pregnan (10*β*,13*β*-Dimethyl-17*β*-ethyl-gonan) aufweisen. Die wichtigsten Vertreter sind Pregnandiol, Progesteron und Pregnenolon. Um die natürlichen Gestagene von den synthetischen Hormonen zu unterscheiden, werden letztere auch als "Progestine" oder "Progestagene" bezeichnet. Ein Beispiel eines Progestagens ist Levonorgestrel, ein synthetisches Gestagen der 2. Generation, welches zur hormonellen Empfängnisverhütung (Kontrazeption) eingesetzt wird. Levonorgestrel wird auch in der Hormonspirale Kyleena® verwendet.

In einer Ausführungsform handelt es sich bei dem intrauterinen Wirkstoffreisetzungssystem um eine Hormonspirale (wie z.B. Mirena®, Kyleena® oder Jaydess®), vorzugsweise um eine Hormonspirale, die Levonorgestrel als Wirkstoff umfasst, wie zum Beispiel das unter dem Markennamen Kyleena® erhältliche Produkt (siehe z.B. https://www.patienteninfo-service.de/a-z-liste/k/kyleenaR-195-mg-intrauterines-wirkstofffreisetzungssystem/). Ein weiteres Beispiel eines bevorzugten intrauterinen Wirkstoffreisetzungssystems ist eines, das Indomethacin und Levonorgestrel als Wirkstoffe umfasst.

Unter dem Begriff "Blutungsverhalten" wird das Auftreten oder Nicht-Auftreten von Regelblutungen bei der Frau verstanden. Parameter, die das Blutungsverhalten charakterisieren können, sind zum Beispiel die Intensität der Blutungen (Blutungsintensität), die Dauer der Blutungen, sowie die Regelmäßigkeit der Blutungen. Die Angabe der Blutungsintensität kann in Form einer Ja-/Nein-Aussage (Blutung/keine Blutung) oder in Form einer dreistufigen Einteilung (keine Blutung/Schmierblutung/Blutung) oder in Form von noch mehr Stufen erfolgen.

Unter dem Begriff "Vorhersage des zukünftigen Blutungsverhaltens" wird eine Aussage darüber verstanden, wie das Blutungsverhalten in der Zukunft aufgrund von Berechnungen sein wird. Die Aussage ist somit mit einer Unsicherheit behaftet. Die Größe der Wahrscheinlichkeit, dass das vorhergesagte Verhalten tatsächlich eintreten wird, kann ermittelt und angegeben werden.

Die Vorhersagen, die auf Basis der vorliegenden Erfindung getroffen werden, erfüllen die Erfordernisse einer triftigen Prognose: Nichttrivialität, Objektivität und Validität.

Die Vorhersage des zukünftigen Blutungsverhaltens erfolgt auf Basis von Prädiktoren.

Unter einem "Prädiktor" wird allgemein eine Variable verstanden, die benutzt wird, um die Werte einer anderen Variable vorherzusagen oder zu modellieren. Im vorliegenden Fall werden Prädiktoren verwendet, um das Blutungsverhalten einer Frau, die ein intrauterines Wirkstofffreisetzungssystem verwendet, vorherzusagen.

Ein Prädiktor im Sinne der vorliegenden Erfindung kann eine (physikalisch) messbare Größe sein, wie zum Beispiel die Anzahl von Tagen innerhalb eines Beobachtungszeitraum, an denen Blutungen aufgetreten sind. Es kann sich aber auch um eine subjektive Größe handeln, wie zum Beispiel die durch die Frau selbst eingeschätzte Intensität einer Blutung.

Ein Prädiktor kann in der Regel mehrere Werte, nachfolgend auch als Prädiktorwerte bezeichnet, aufweisen. Werte können Zahlen mit einer Maßeinheit sein, wie zum Beispiel das Alter einer Frau (z.B. 32 Jahre). Werte können auch Zahlen ohne Maßeinheit sein, wie zum Beispiel die Zahl der Schwangerschaften vor Einsetzen eines intrauterinen Wirkstofffreisetzungssystems. Werte können auch in Form von Begriffen ausgedrückt werden; zum Beispiel kann die selbst eingeschätzte Intensität einer Blutung mit einem der Begriffe: "sehr schwach", "schwach", "stark", "sehr stark" ausgedrückt werden.

Mögliche Prädiktoren sind Informationen zu der Frau, die ein intrauterines Wirkstofffreisetzungssystem trägt, wie z.B.:
- das Alter der Frau,
- die Verhütungsmethode, die die Frau vor dem Einsatz des intrauterinen Wirkstofffreisetzungssystems verwendet hat,
- die Zahl der bisherigen Schwangerschaften/Geburten der Frau,
- die (regelmäßige) Einnahme von Tabletten und/oder Suchtmitteln (z.B. Zigaretten),
- Angaben zum Blutungsverhalten in einem definierten Zeitraum (z.B. drei Monate) vor Einsetzen eines intrauterinen Wirkstofffreisetzungssystems.

Auch genetische Informationen, Informationen über die aufgenommene Nahrung, die körperliche Fitness, und andere Informationen über den Gesundheitszustand einer Frau sind als Prädiktoren denkbar.

Prädiktoren lassen sich beispielsweise in Studien ermitteln oder durch Analyse von laufenden/abgeschlossenen Studien identifizieren.

Bevorzugte Prädiktoren sind Informationen zum Blutungsverhalten innerhalb einer Beobachtungszeitspanne nach dem Einsetzen des intrauterinen Wirkstofffreisetzungssystems, dazu zählen:
- Zeitpunkte, an denen Blutungen innerhalb des Beobachtungszeitraumes aufgetreten sind,
- Zeitspannen, in denen die Blutungen andauerten,
- Intensitäten der Blutungen
- Gesamtzahl der Blutungstage
- Gesamtzahl der Spottingtage
- Regularität der Blutung (bzgl. Dauer und Intensität)
- Anzahl von Blutungs- oder Spottingepisoden.

Die Beobachtungszeitspanne beginnt mit dem Einsetzen des intrauterinen Wirkstofffreisetzungssystems und dauert mindestens 60 Tage. Vorzugsweise liegt die Dauer der Beobachtungszeitspanne im Bereich von 61 Tagen bis 120 Tagen. Besonders bevorzugt liegt die Dauer der Beobachtungszeitspanne im Bereich von 70 Tagen bis 110 Tagen, noch mehr bevorzugt im Bereich von 80 bis 100 Tagen, noch mehr bevorzugt im Bereich von 85 bis 95 Tagen, ganz besonders bevorzugt im Bereich von 88 bis 90 Tagen.

Es hat sich gezeigt, dass das Blutungsverhalten innerhalb einer Beobachtungszeitspanne beginnend mit dem Einsetzen des intrauterinen Wirkstofffreisetzungssystems Prädiktoren bereitstellt, mit deren Hilfe sich das zukünftige Blutungsverhalten, das heißt, das Blutungsverhalten in einer Zeitspanne, die an die Beobachtungszeitspanne anschließt (Vorhersagezeitspanne), vorhersagen lässt. Mit Hilfe der vorliegenden Erfindung kann ein Arzt und/oder die Frau spätestens nach Ablauf des Beobachtungszeitraums ermitteln, wie sich das Blutungsverhalten in der Zukunft wahrscheinlich einstellen wird, so dass der Arzt und die Frau darüber diskutieren können, ob es sinnvoll ist, die Behandlung beizubehalten.

Die Beobachtungszeitspanne erstreckt sich daher in einer bevorzugten Ausführungsform vom Zeitpunkt des Einsetzens des intrauterinen Wirkstofffreisetzungssystems bis zum Zeitpunkt einer ersten, zweiten oder dritten Nachuntersuchung z.B. durch einen Arzt (engl. *health professional, health practitioner, healthcare provider*). Es ist denkbar, dass eine Frau, der intrauterines Wirkstofffreisetzungssystem eingesetzt worden ist, vier Wochen oder fünf Wochen oder sechs Wochen oder sieben Wochen oder acht Wochen oder neun Wochen oder 10 Tage oder 20 Tage oder 30 Tage oder 40 Tage oder 50 Tage oder 60 Tage oder 70 Tage oder 80 Tage oder 90 Tage oder 100 Tage oder 110 Tage oder 120 Tage oder einem anderen Zeitraum nach dem Einsetzen des intrauterinen Wirkstofffreisetzungssystems zu einer Nachuntersuchung gebeten wird.

Zur Ermittlung und/oder Sammlung von Prädiktorenwerten wird vorzugsweise ein Computersystem verwendet. Es ist auch denkbar, mehrere Computersysteme zu verwenden.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computer von heute werden häufig in Desktop-PCs, Portable PCs, Laptops, Notebooks, Netbooks und Tablet-PCs und so genannte Handhelds (z.B. Smartphone) unterteilt.

Vorzugsweise wird ein mobiles Computersystem wie ein Smartphone, ein Laptop, ein Tablet-Computer oder eine Smartwatch zur Erfassung und/oder Ermittlung und/oder Sammlung von Prädiktorenwerten verwendet. Es kann jedoch auch ein stationäres Computersystem (z.B. ein Desktop-Computer oder ein Terminal oder dergleichen) verwendet werden. Das Computersystem, das zur Erfassung/Ermittlung/Sammlung von Prädiktorenwerten eingesetzt wird, wird in dieser Beschreibung auch als "das erste Computersystem" bezeichnet. Auch die Verwendung von mehreren ersten Computersystemen zur Erfassung/Ermittlung/Sammlung von Prädiktorenwerten ist denkbar.

Das erste Computersystem wird üblicherweise durch die Frau, für die das zukünftige Blutungsverhalten vorausgesagt werden soll, bedient. Sie wird hier auch als "Nutzerin" bezeichnet. Der Begriff "Nutzerin" bezieht sich dabei sowohl auf die Benutzung des ersten Computersystems als auch auf die Benutzung des erfindungsgemäßen Computerprogrammprodukts.

In einer bevorzugten Ausführungsform werden die Prädiktorenwerte über eine grafische Benutzerschnittstelle (engl. *graphical user interface,* GUI) erfasst. Die Prädiktorenwerte können zum Beispiel mittels einer (virtuellen) Tastatur in Form von Text/Zahlen eingegeben werden. Denkbar ist auch die Auswahl von Prädiktorenwerten aus einer oder mehreren virtuellen Listen, die auf einem Bildschirm angezeigt werden. Auch eine Eingabe von Prädiktorenwerten über ein Mikrofon mittels Spracheingabe ist denkbar.

Es gibt Prädiktoren, die mit einem oder mehreren Zeitpunkten verbunden sind. Zum Beispiel ist eine im Beobachtungszeitraum auftretende Blutung mit dem Zeitpunkt verbunden, an dem die Blutung aufgetreten ist. Die Zeitspanne der Blutung ist mit dem Zeitpunkt verbunden, an dem die Blutung aufgetreten ist und sie ist mit dem Zeitpunkt verbunden, an dem die Blutung verschwunden ist. Vorzugsweise werden zumindest einige Werte von zumindest einigen Prädiktoren, die mit einem oder mehreren Zeitpunkten verbunden sind, über eine grafische Benutzerschnittstelle in einen virtuellen Kalender eingetragen. Die Eintragung erfolgt vorzugsweise durch die Nutzerin, kann aber auch durch eine andere Person wie beispielsweise Pflegepersonal oder einen Arzt erfolgen.

In einer bevorzugten Ausführungsform wird zur Erfassung von Prädiktorenwerten während des Beobachtungszeitraumes ein virtueller Kalender geführt, in den der Zeitpunkt des Auftretens von Blutungen, die Zeitspanne der Blutungen und optional die Intensität der Blutungen eingetragen werden.

In einer bevorzugten Ausführungsform werden von der Nutzerin mit Hilfe des ersten Computersystems diejenigen Tage innerhalb der Beobachtungszeitspanne festgehalten, an denen eine Blutung oder eine Schmierblutung aufgetreten ist, und ob es sich um eine Blutung oder eine Schmierblutung gehandelt hat.

Es ist auch denkbar, dass Prädiktorenwerte abgefragt werden. Es ist zum Beispiel denkbar, dass das erfindungsgemäße Computerprogrammprodukt über eine "Alarmfunktion" verfügt, die bei Eintritt eines Ereignisses und/oder bei Erreichen eines Zeitpunktes eine Nachricht an die Nutzerin (oder eine andere Person wie z.B. Pflegepersonal) übermittelt. Es ist zum Beispiel denkbar, dass das Computerprogrammprodukt so konfiguriert ist, dass es bei Erreichen oder Annähern eines Zeitpunktes, bei dem aufgrund von Wahrscheinlichkeitsberechnungen mit dem Eintritt der Regelblutung zu rechnen ist, eine Nachricht an die Nutzerin übermittelt und die Nutzerin befragt, ob eine Regelblutung aufgetreten ist. Die Nutzerin kann den Eintritt der Regelblutung durch Drücken eines (virtuellen) Knopfes auf der grafischen Benutzeroberfläche bestätigen oder verneinen. Sie kann ferner Angaben zur Stärke der Blutung machen.

Es ist ferner denkbar, dass das Erfassen von allen oder einem Teil der Prädiktorenwerte durch einen oder mehrere Sensoren unterstützt wird. Es ist zum Beispiel denkbar, dass ein Sensor das Auftreten und/oder Verschwinden von Regelblutungen und/oder ihre Intensität erfasst.

Es ist auch denkbar, einen Sensor oder mehrere Sensoren zu verwenden, die durch die Nutzerin bedient werden. Als Beispiel sei ein Sensor aufgeführt, den eine Nutzerin betätigt, wenn sie aufgrund der eintretenden Regelblutung Produkte der Damenhygiene (z.B. Binde, Tampon) nutzt (ein Beispiel eines solchen Sensors ist der von Amazon vertriebene Dash-Button). Es ist auch denkbar, dass an dem Produkt der Damenhygiene oder in dessen Nähe ein Sensor angebracht ist, den die Nutzerin betätigt, wenn sie eine Produkteinheit einer Verpackung entnimmt. Denkbar ist auch, dass ein Sensor an einer Verpackung angebracht ist, der automatisch erfasst, wenn die Verpackung geöffnet wird. Das vom Sensor an das erste Computersystem übertragene Signal bedeutet dann, dass eine Blutung aufgetreten ist und wird als solche Information vom ersten Computersystem erfasst.

Der Sensor kann so ausgestaltet sein, dass er bei Bedienung und/oder bei der Erfassung eines Signals eine Mitteilung über eine kurzreichweitige Verbindung (z.B. mittels Funk über einen Übertragungsstandard wie Bluetooth oder Zigbee oder dergleichen) an eine Empfangseinrichtung übermittelt. Die Empfangseinrichtung kann an ein Netzwerk (z.B. Internet) angeschlossen sein und so die Mitteilung an weitere an das Netzwerk angeschlossene Einheiten (z.B. das erste Computersystem) weiterleiten.

Die erfassten Prädiktorenwerte werden verwendet, um eine Vorhersage über das zukünftige Blutungsverhalten der Nutzerin zu treffen. Dazu wird ein Vorhersagemodell verwendet. Es ist auch denkbar, eine Vorhersage auf Basis von mehreren Vorhersagemodellen zu treffen; zur sprachlichen Vereinfachung wird im Folgenden jedoch von einem Vorhersagemodell ausgegangen. Das Vorhersagemodell kann zum Beispiel auf Basis einer Studie ermittelt werden, an der üblicherweise eine Vielzahl von Frauen, denen ein intrauterines Wirkstofffreisetzungssystem eingesetzt worden ist, teilnimmt. Es ist denkbar, dass auf Basis der Daten, die in einer solchen Studie gewonnen werden, ein künstliches neuronales Netz oder ein anderes System der künstlichen Intelligenz trainiert wird (Maschinelles Lernen). Das trainierte System kann dann als Vorhersagemodell eingesetzt werden.

In einer bevorzugten Ausführungsform wird ein Vorhersagemodell auf Basis eines Random-Forrest-Ansatzes erstellt.

In einer anderen bevorzugten Ausführungsform wird ein Vorhersagemodell auf Basis eines Regressionsbaumes (engl. *regression tree*) erstellt.

Die Vorhersage des zukünftigen Blutungsverhaltens erfolgt vorzugsweise für eine Vorhersagezeitspanne. Das bedeutet, dass auf Basis des Blutungsverhaltens in der Beobachtungszeitspanne und ggf. auf Basis weiterer Prädiktoren das Blutungsverhalten in einer Vorhersagezeitspanne vorhergesagt wird, die zeitlich unmittelbar auf die Beobachtungszeitspanne folgt.

Im Rahmen der Arbeiten, die dieser Erfindung zu Grunde liegen, wurde unter anderem eine Vorhersagezeitspanne von 180 Tagen verwendet. Wenn die Beobachtungszeitspanne also beispielsweise 90 Tage beträgt, erfolgt eine Vorhersage des Blutungsverhaltens für die Zeitspanne von 91 bis 270 Tage nach Einsetzen des intrauterinen Wirkstofffreisetzungssystems. Denkbar sind jedoch auch andere Dauern der Vorhersagezeitspanne. Für die Nutzerin ist die Vorhersagezeitspanne eher unbedeutend, denn sie wird wahrscheinlich erwarten, dass das Blutungsverhalten nach der Vorhersagezeitspanne unter normalen Bedingungen (z.B. kein Auftreten von Erkrankungen oder Entnahme des intrauterinen Wirkstoffsystems oder dergleichen) im Wesentlichen dem Verhalten in der Vorhersagezeitspanne entspricht. Für das Erstellen des Vorhersagemodells ist eine Festlegung der Vorhersagezeitspanne insofern von Bedeutung als die Identifikation von Klassen für diejenigen Daten erfolgt, die in dieser Vorhersagezeitspanne erhoben worden sind (insbesondere das Blutungsverhalten).

Die Vorhersagezeitspanne sollte eine Dauer aufweisen, die mehr als eine Monatsperiode (ungefähr 28 Tage) umfasst; insbesondere, um in Daten zum Blutungsverhalten eine Regelmäßigkeit hinsichtlich des Auftretens der Blutungen identifizieren zu können. Je länger die Vorhersagezeitspanne ist, desto mehr Daten liegen zur Modellbildung vor und desto zukunftsorientierter ist der Informationsgehalt für die Frau. Auf der anderen Seite steigt mit einer längeren Vorhersagezeitspanne die Ungenauigkeit der Vorhersage. Darüber hinaus lassen sich längere Vorhersagezeitspannen schwieriger durch simple Parameter zusammenfassen, da so ggf. Muster übersehen werden. Beispielsweise könnte es Patienten geben, die in der ersten Hälfte des Zeitraums noch sehr häufig bluten, in der 2. Hälfte jedoch kaum noch. Weiter könnte es Patienten geben, die über den kompletten Zeitraum "mittelhäufig" bluten. Je länger die Vorhersagezeitspanne ist, desto wahrscheinlicher ist das Auftreten von Nebeneffekten (z.B. Krankheiten), die eine Klassenbildung erschweren können. Zudem ist die Erfassung von Daten über einen längeren Zeitraum aufwändiger und teurer als für einen kürzeren Zeitraum. Es hat sich gezeigt, dass eine Vorhersagezeitspanne von 180 Tagen sehr gut geeignet ist, eine Klassenbildung vorzunehmen und ein Vorhersagemodell zu erzeugen. Denkbar ist aber auch eine Vorhersagezeitspanne mit einer anderen Dauer.

Vorzugsweise erfolgt die Vorhersage auf Basis eines Klassifikationsmodells. Das Klassifikationsmodell ordnet die Frau anhand der erfassten Prädiktoren einer von mehreren Blutungsverhaltensklassen zu. Die Blutungsverhaltensklassen unterscheiden sich hinsichtlich zu erwartender Blutungsintensitäten in der Vorhersagezeitspanne. Frauen, die einer der Blutungsverhaltensklassen zugeordnet sind, zeigen ein ähnliches Blutungsverhalten.

Die Klasse, zu der eine Frau zugeordnet wird, gibt dann mit einer bestimmbaren Wahrscheinlichkeit an, wie die Regelblutungen nach der Beobachtungszeitspanne (in der Vorhersagezeitspanne) sein werden (zeitliches Auftreten, Dauer, Intensität). Die Zuordnung der Frau zu einer Klasse von Frauen mit einem bestimmten Blutungsmuster wird auch als Klassifizierung bezeichnet.

Die Zahl der Klassen mit jeweils ähnlichem Blutungsverhalten beträgt vorzugsweise zwei, drei, vier, fünf, sechs, sieben oder acht.

Bei einer Zahl von vier Klassen könnte zum Beispiel eine Zuordnung einer Frau zu einer der folgenden Klassen vorgenommen werden:
Klasse I: Amenorrhoe
Klasse II: hauptsächlich Schmierblutungen (engl. *spotting*)
Klasse III: reguläre Blutungen
Klasse IV: irreguläre Blutungen

Die Begriffe "Blutungen" und "Schmierblutungen" werden vorzugsweise entsprechend der Definition der World Health Organisation (WHO) verstanden (Belsey E.M. et al.: The analysis of vaginal bleeding patterns induced by fertility regulating methods, Contraception 1986, 34, 253-260):
Blutung: vaginaler Blutverlust, die des Einsatzes eines Hygieneerzeugnisses wie beispielsweise einer Binde oder eines Tampons bedarf
Schmierblutung: vaginaler Blutverlust, für den kein Einsatz eines Hygieneerzeugnisses notwendig ist
Blutungstag: ein Tag, an dem eine Blutung aufgetreten ist
Schmierblutungstag: ein Tag, an dem eine Schmierblutung aufgetreten ist

Andere Klassenbeschreibungen/ Klassendefinitionen sind denkbar.

In einer besonders bevorzugten Ausführungsform wird eine Klassifizierung in drei Klassen vorgenommen:
Klasse (20): Vorwiegendes Blutungsmuster: Amenorrhoe
Klasse (30): Vorwiegendes Blutungsmuster: Schmierblutungen
Klasse (40): Vorwiegendes Blutungsmuster: Blutungen

Die Definition der Klassen kann beispielsweise über die relativen Häufigkeiten der Blutungs- bzw. der Schmierblutungstage erfolgen.

Tabelle 1 zeigt ein Beispiel für die Definition der oben genannten Klassen (20), (30) und (40).

**Tab. 1: Beispielhafte Definition von Blutungsverhaltenklassen**

| | |
|---|---|
| Klasse (20): Vorwiegendes | weniger als 5% Schmierblutungstage und |
| Blutungsmuster: Amenorrhoe | weniger als 1% Blutungstage |
| Klasse (30): Vorwiegendes | nicht Klasse (20) zugehörig und |
| Blutungsmuster: Schmierblutungen | weniger als 5% Blutungstage |
| Klasse (40): Vorwiegendes | nicht Klasse (20) und nicht Klasse (30) zugehörig |
| Blutungsmuster: Blutungen | |

In einer bevorzugten Ausführungsform erfolgt für diejenigen Frauen, für die Blutungen und/oder Schmierblutungen vorhergesagt werden, zusätzlich eine Vorhersage hinsichtlich der Regelmäßigkeit der Blutungen.

Eine solche Aussage zur Regelmäßigkeit von Blutungen kann beispielsweise sein, dass für eine Frau eine eher regelmäßige Blutung oder eine eher unregelmäßige Blutung vorausgesagt wird. Ebenso ist es denkbar, dass man die Regelmäßigkeit mit einem Wert angibt, z.B. 100% für eine absolute regelmäßige Periode, bei der zwischen dem Beginn einer Periode und dem Beginn der nachfolgenden Periode immer genau die gleiche Zahl von Tagen liegt, und 0% für eine Periode, die keine Regelmäßigkeit erkennen lässt (zufällige Blutungen). Denkbar ist ferner ein Farbcode, der zum Beispiel von grün (regelmäßig) über gelb (leichte Schwankungen von 3 bis 7 Tagen) bis rot (unregelmäßig, Schwankungen von mehr als 7 Tagen) variiert.

Denkbar ist auch eine Aussage über die zu erwartende Periodendauer und/oder die mittlere Schwankungsbreite der Periodendauer.

In einer bevorzugten Ausführungsform erfolgt eine Aussage zur Wahrscheinlichkeit einer Regelmäßigkeit auf Basis eines logistischen Regressionsmodells.

Grundlage für die Vorhersage zur Regelmäßigkeit ist vorzugsweise eine berechnete Periodenlänge, die auf Basis der Blutungstage im Beobachtungszeitraum oder im Vorhersagezeitraum mit Hilfe einer Autokorrelationsfunktion berechnet wird. Üblicherweise schwankt die Periodenlänge (auch Zykluslänge oder Zyklusdauer genannt) zwischen 21 und 35 Tagen. Die Blutungsintensitäten als Funktion der Zeit stellen eine Blutungszeitfunktion dar, die sich bei einer absoluten Regelmäßigkeit üblicherweise alle 21 bis 35 Tage wiederholt. Verschiebt man also diese Blutungszeitfunktion um den Betrag der Periodenlänge, kommt die verschobene Blutungszeitfunktion mit der unverschobenen Blutungszeitfunktion zur Deckung. Verschiebt man die Blutungszeitfunktion mehrfach um jeweils eine definierte Zeitspanne (z.B. einen Tag) und berechnet jeweils den Wert der Autokorrelation zwischen der verschobenen und der unverschobenen Funktion, so ergibt sich ein maximaler Wert der Autokorrelation bei einer Verschiebung, die der Periodenlänge entspricht. Auf diese Weise lässt sich eine Periodenlänge (Wert der Verschiebung, bei der ein Maximum der Autokorrelation auftritt) berechnen. Die Größe des Wertes der Autokorrelation im Maximum kann ferner als Parameter für die Regelmäßigkeit verwendet werden. Je größer der Wert ist, desto regelmäßiger ist die Periode.

Es ist aber auch denkbar, die Regelmäßigkeit auf Basis anderer und/oder weiterer Parameter zu bestimmen.

In einer Ausführungsform wird die Blutungszeitfunktion zwei Zeitreihenmodellen zugeführt; eines der Modelle verfügt über einen saisonalen Parameter (berechnete Periodenlänge); das andere Modell kommt ohne einen solchen Parameter aus; dasjenige Zeitreihenmodell, welches für die Blutungszeitfunktion ein höheres Informationskriterium ergibt (z.B. Akaikes Informationskriterium), beschreibt die Blutungszeitfunktion besser; ist es dasjenige mit saisonalem Parameter, so liegt eine eher regelmäßige Periode vor; ist es dasjenige ohne saisonalem Parameter, so liegt eine eher unregelmäßige Periode vor.

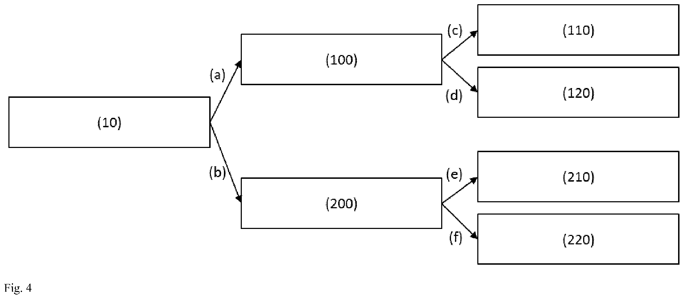

In einer weiteren bevorzugten Ausführungsform erfolgt die Vorhersage des Blutungsverhaltens auf Basis eines Regressionsbaumes. Figur 4 zeigt beispielhaft einen erfolgreichen Ansatz hierzu. Anhand der Zahl der Blutungstage in der Beobachtungszeitspanne lassen sich Frauen in zwei Gruppen einteilen; eine Gruppe mit mehr Blutungstagen in der Vorhersagezeitspanne und eine Gruppe mit weniger Blutungstagen in der Vorhersagezeitspanne. Die Gruppe der Frauen mit mehr Blutungstagen lässt sich ferner auf Basis der Werte der Autokorrelation der Blutungszeitfunktion in der Beobachtungszeitspanne in zwei weitere Gruppen einteilen; eine Gruppe, bei denen die Blutungen in der Vorhersagezeitspanne eher unregelmäßig sind und eine Gruppe, bei denen die Blutungen in der Vorhersagezeitspanne eher regelmäßig sind. Die Gruppe der Frauen mit weniger Blutungstagen lässt sich auf Basis der Zahl der Schmierblutungstage in der Beobachtungszeitspanne in zwei Gruppen einteilen; eine Gruppe mit mehr Schmierblutungstagen in der Vorhersagezeitspanne und eine Gruppe mit weniger Schmierblutungstagen in der Vorhersagezeitspanne.

In einer weiteren Ausführungsform der vorliegenden Erfindung wird auf Basis von Prädiktoren ein für die jeweilige Frau individuelles zukünftiges Blutungsverhalten ermittelt und angezeigt. Dazu wird die Frau zunächst auf Basis von Prädiktoren einer Klasse zugeordnet, es wird das für die Klasse zu erwartende Blutungsverhalten ermittelt, und es wird das ermittelte Blutungsverhalten individuell an die Frau angepasst. Eine solche Anpassung kann zum Beispiel darin bestehen, die Zeitpunkte von zu erwartenden Blutungen der Klasse an das individuelle bisherige Blutungsverhalten der Frau anzupassen. Bei einer solchen Anpassung können beispielsweise der Zeitpunkt der letzten Blutung und die mittlere Periodendauer einer Frau verwendet werden, um die Zeitpunkte und Intensitäten der zukünftigen Blutungen der Frau individueller vorherzusagen.

Die Vorhersage des zukünftigen Blutungsverhaltens erfolgt erfindungsgemäß durch eine Vorhersageeinheit.

Die Vorhersageeinheit ist eine Einheit, die Prädiktorenwerte entgegennimmt, sie einem Vorhersagemodell zuführt (oder mehreren Vorhersagenmodellen zuführt) und mit Hilfe des Vorhersagemodells das zukünftige Blutungsverhalten einer Nutzerin ermittelt.

Die Vorhersageeinheit empfängt die Prädiktorenwerte (oder zumindest einen Teil der Prädiktorenwerte) vorzugsweise direkt von der Erfassungseinheit, führt die Vorhersage (z.B. eine Klassifizierung) durch und übermittelt das Ergebnis der Vorhersage (z.B. der Klassifizierung) an eine Ausgabeeinheit.

Die Vorhersageeinheit ist vorzugsweise Bestandteil eines Computersystems. In einer Ausführungsform der vorliegenden Erfindung ist die Vorhersageeinheit ein Bestandteil desjenigen Computersystems, mit dem Prädiktorenwerte erfasst worden sind, d.h. des hier so genannten "ersten Computersystems". Das erste Computersystem kann ein mobiles Computersystem (Laptop, Tablet-Computer, Smartwatch, Smartphone oder dergleichen) oder ein stationäres Computersystem (Desktop-Computer, Terminal oder dergleichen) sein. Vorzugsweise handelt es sich um ein mobiles Computersystem.

Die Vorhersageeinheit kann jedoch auch Bestandteil eines Computersystems sein, das nicht mit dem ersten Computersystem identisch ist. Ein solches separates Computersystem, das eine Vorhersageeinheit umfasst, wird hier auch als "zweites Computersystem" bezeichnet. Liegen ein erstes Computersystem umfassend eine Erfassungseinheit und ein separates zweites Computersystem umfassend eine Vorhersageeinheit vor, so müssen die Prädiktorenwerte vom ersten Computersystem an das zweite Computersystem übermittelt werden, um eine Vorhersage (z.B. Klassifizierung) vornehmen zu können. Eine solche Datenübertragung kann beispielsweise mit Hilfe eines Datenträgers erfolgen, auf dem die Daten vom ersten Computersystem gespeichert werden und von dem die Daten vom zweiten Computersystem gelesen (in einen Arbeitsspeicher geladen) werden. Beispiele für einen solchen Datenträger sind Disketten, Compact Disks, Festplatten, USB-Sticks, Speicherkarten und dergleichen.

Vorzugsweise erfolgt die Übertragung von Daten (z.B. Prädiktorenwerten) von dem ersten Computersystem an das zweite Computersystem (und gegebenenfalls auch umgekehrt vom zweiten Computersystem an das erste Computersystem) über ein Netzwerk, vorzugsweise über das Internet und/oder über ein Mobilfunknetz.

Ein Grund für die Aufteilung von Erfassung und Vorhersage auf zwei verschiedene Computersysteme könnte sein, dass für die Vorhersage eines Blutungsverhaltens einer Nutzerin mehr Rechenleistung notwendig ist als beispielsweise zur Erfassung der Prädiktorenwerte. In einem solchen Fall könnte für die Erfassung der Prädiktorenwerte ein vergleichsweise einfaches und damit vergleichsweise günstiges erstes Computersystem verwendet werden, wie beispielsweise ein Smartphone. Ein Smartphone als Erfassungseinheit hätte zudem den Vorteil, dass es üblicherweise ständiger Begleiter von vielen Nutzerinnen ist, so dass eine Nutzerin bei einer unverhofft auftretenden oder auch erwarteten Blutung entsprechende Daten in das Smartphone einpflegen kann. Das Smartphone könnte so konfiguriert sein, dass es z.B. bei Vorliegen von einer Mindestdatenmenge die Daten (Prädiktorenwerte) über das Mobilfunknetz oder über ein WLAN-Netz und das Internet an ein separates zweites Computersystem mit einer vergleichsweise hohen Rechenleistung übermittelt. Auf dem zweiten Computersystem erfolgen dann die Auswertung der Prädiktorenwerte und die Vorhersage des Blutungsverhaltens. Ein solches System mit separaten Computersystemen hätte ferner den Vorteil, dass das Klassifikationsmodell fortwährend verbessert und aktualisiert werden könnte, ohne dass eine Aktualisierung der Erfassungssoftware auf dem ersten Computersystem notwendig wäre. Das zweite Computersystem könnte zudem die Vorhersagen vieler Nutzerinnen übernehmen, die mit ihren ersten Computersystemen über ein Netzwerk mit dem zweiten (zentralen) Computersystem verbunden sind.

Es ist denkbar, dass eine Vorhersage des Blutungsverhalten erst dann erfolgt, wenn "alle vorgesehenen" Prädiktorenwerte erfasst worden sind. Es ist zum Beispiel denkbar, dass mit Hilfe des ersten Computersystems Prädiktorenwerte dadurch erfasst werden, dass Prädiktorenwerte abgefragt werden und/oder Prädiktorenwerte in Felder einer grafischen Benutzeroberfläche eingetragen werden und die so erfassten Prädiktorenwerte erst dann der Vorhersage zugeführt werden, wenn alle Prädiktorenwerte abgefragt und eingetragen sind.

Es ist aber auch denkbar, dass fortwährend Prädiktorenwerte erfasst und der Vorhersage zugeführt werden, um eine stetig verbesserte (zunehmend genaue) Vorhersage zu erzeugen.

Auch Vorgehensweisen zwischen den beiden genannten Vorgehensweisen sind denkbar; eine Vorhersage kann zum Beispiel dann erfolgen, wenn eine Mindestmenge und/oder eine definierte Menge an Prädiktorenwerten erfasst worden ist; die Vorhersage kann aktualisiert werden, wenn weitere Prädiktorenwerte vorliegen. Die Mindestmenge an Prädiktorenwerten kann beispielsweise dadurch definiert sein, dass erst ab dieser Mindestmenge eine Vorhersage getroffen werden kann, deren Wahrscheinlichkeit des Eintretens oberhalb eines definierten Schwellenwerts (z.B. 50 %) liegt.

In einer bevorzugten Ausführungsform wird ein erster Satz an Prädiktorenwerten erfasst und eine erste Vorhersage getroffen. Bevorzugt handelt es sich bei diesem ersten Satz an Prädiktorenwerten um Werte, die zum Zeitpunkt des Einsetzens eines intrauterinen Wirkstofffreisetzungssystems zur Verfügung stehen/bekannt sind. Nach der ersten Vorhersage werden weitere Prädiktorenwerte erfasst, vorzugsweise immer dann, wenn sie (zeitlich) anfallen. Diese weiteren Prädiktorenwerte werden dann fortwährend der Vorhersageeinheit zugeführt oder zu definierten Zeitpunkten oder bei Eintritt definierter Ereignisse der Vorhersageeinheit zugeführt, um eine zweite, dritte und/oder weitere Vorhersagen zu erzeugen. Üblicherweise wird eine Vorhersage mit zunehmender Menge an vorliegenden Prädiktorenwerten besser (genauer, mit weniger Unsicherheit behaftet).

Nachdem das zukünftige Blutungsverhalten einer Nutzerin ermittelt worden ist, erfolgt eine Ausgabe von Informationen über das ermittelte zukünftige Blutungsverhalten an die Nutzerin und/oder an eine andere Person, die einen Bezug zu der Nutzerin hat, wie beispielsweise ein Angehöriger, ein Arzt und/oder Pflegepersonal.

Die Ausgabe der Informationen erfolgt vorzugsweise über einen Bildschirm (z.B. als Textbotschaft und/oder als Grafik) und/oder über Lautsprecher (z.B. als Sprachnachricht) des ersten Computersystems. Es ist aber auch denkbar, dass die Ausgabe auf dem zweiten Computersystem erfolgt, zum Beispiel dann, wenn das zweite Computersystem durch einen Arzt oder eine vergleichbare Person bedient wird.

Die Ausgabe kann folgende Informationen umfassen:
- Intensität von zukünftigen Regelblutungen und/oder
- Häufigkeit von zukünftigen Regelblutungen und/oder
- Dauer von zukünftigen Regelblutungen und/oder
- Zugehörigkeit zu einer definierten Klasse von Frauen mit einem ähnlichen Blutungsverhalten und/oder
- Regelmäßigkeit von zukünftigen Regelblutungen.

In einer bevorzugten Ausführungsform werden zukünftig zu erwartende Blutungen und deren erwartete Intensitäten in einen virtuellen Kalender eingetragen, so dass die Nutzerin in dem Kalender ablesen kann, wann eine Blutung auftreten wird und wie stark sie wahrscheinlich sein wird. Vorzugsweise ist der Kalender mit einer Alarmfunktion ausgestattet, die die Nutzerin vor dem Eintreten einer Blutung daran erinnert, zum Beispiel 72 und/oder 60 und/oder 48 und/oder 36 und/oder 24 und/oder 12 und/oder 6 und/oder 2 Stunden vorher und/oder zu einem anderen Zeitpunkt.

In einer bevorzugten Ausführungsform wird zusätzlich eine Angabe über die Genauigkeit der Vorhersage ausgegeben. Dies gilt insbesondere für den Fall, dass mehrere Vorhersagen erzeugt werden, zum Beispiel weil mit der Zeit zunehmend mehr Prädiktorenwerte zur Verfügung stehen und erfasst werden. Die Ausgabe über die Genauigkeit kann zum Beispiel in Form einer Angabe einer Wahrscheinlichkeit, mit der die Vorhersage eintritt, erfolgen. Die Angabe kann mit grafischen Mitteln, zum Beispiel farblich, unterstützt werden. Zum Beispiel kann eine Angabe zur Wahrscheinlichkeit des Eintritts der Vorhersage mit zunehmender Wahrscheinlichkeit in einem zunehmend dunkleren Farb- oder Grauton dargestellt sein.

Das erfindungsgemäße System kann auch so ausgestaltet sein, dass die Vorhersage über das zukünftige Blutungsverhalten einer Frau gegenüber einem Arzt angezeigt wird. Der Begriff "Arzt" wird in dieser Beschreibung als Sammelbegriff für Menschen verwendet, die sich beruflich mit der Gesundheit von Menschen beschäftigen.

Es ist zum Beispiel denkbar, dass eine Frau mit Hilfe eines mobilen ersten Computersystems zumindest das Blutungsverhalten in einem Beobachtungszeitraum nach Einsetzen eines intrauterinen Wirkstofffreisetzungssystems erfasst. Bei einer Nachuntersuchung überträgt der Arzt und/oder die Frau die gesammelten Daten auf ein zweites Computersystem, auf dem auf Basis der gesammelten Daten und ggf. weiterer Daten über die Nutzerin eine Vorhersage über das zukünftige Blutungsverhalten der Frau erstellt und dem Arzt angezeigt wird, so dass er die Frau beraten kann und er zusammen mit der Frau entscheiden kann, ob Maßnahmen getroffen werden sollten.

Gegenstand der vorliegenden Erfindung ist auch ein Computerprogrammprodukt. Das Computerprogrammprodukt umfasst einen Datenträger, auf dem ein Computerprogramm gespeichert ist, das in den Arbeitsspeicher eines Computersystems geladen werden kann und dort das Computersystem dazu veranlasst, einen oder mehrere Schritte des erfindungsgemäßen Verfahrens ausführen.

Die vorliegende Erfindung soll auch die Situation erfassen, dass Funktionalitäten des Computerprogramms auf mehrere Computersysteme verteilt sind. So ist es wie bereits beschrieben denkbar, dass die Erfassungseinheit Bestandteil eines Computersystems ist, während die Vorhersageeinheit Bestandteil eines anderen (separaten) Computersystems ist. Genauso ist es denkbar, dass für die Erfassung und/oder die Vorhersage mehrere Computersysteme verwendet werden. Auch die Ausgabeeinheit kann Bestandteil eines separaten Computersystems sein.

Die Erfindung wird nachfolgend anhand von Figuren und bevorzugten Ausführungsformen näher erläutert, ohne die Erfindung auf die in den Figuren gezeigten Merkmale oder die beschriebenen Ausführungsformen beschränken zu wollen.

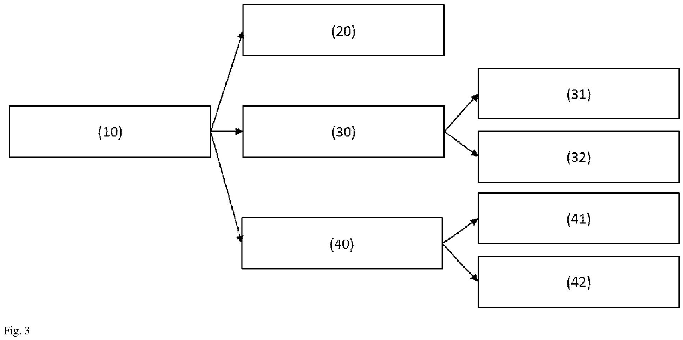

Es zeigen:

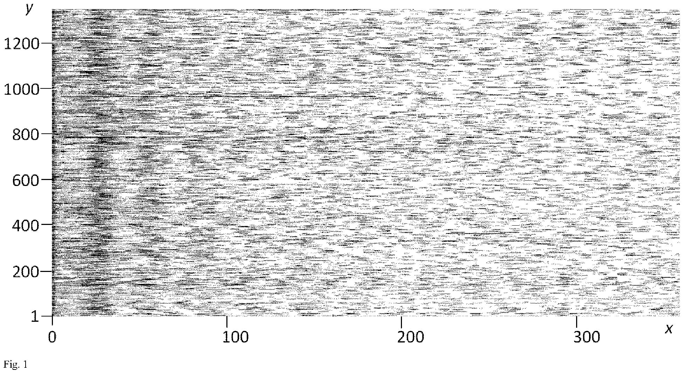

Fig. 1 zeigt das Ergebnis einer Studie mit 1351 Frauen, denen am Tag 0 die unter dem Markennamen Kyleena® vertriebene Hormonspirale eingesetzt worden ist.
Es ist für die 1351 Frauen (Ordinate *y*) dargestellt, an welchen von 360 Tagen (Abszisse x) nach Einsetzen der Hormonspirale eine Blutung aufgetreten ist und wie stark diese Blutung jeweils war (Blutungsdaten; zunehmender Grauwert bei zunehmender Blutungsintensität).
Fig. 2 zeigt dieselbe Grafik wie Fig. 1, in der eine Vorhersagezeitspanne (1) und eine Beobachtungszeitspanne (2) eingezeichnet sind.
Die Blutungsdaten in der Zeit von Tag 91 bis Tag 270 wurden näher untersucht, um Klassen mit ähnlichem Blutungsverhalten zu identifizieren. Dieser Bereich der Blutungsdaten (von Tag 91 bis Tag 270) wird in dieser Beschreibung auch als Vorhersagezeitspanne (1) bezeichnet. Es wurden Klassen auf Basis von Blutungs- und Schmierblutungstagen definiert (siehe Figur 3, Klassen (20), (30), (40) sowie die dazugehörige Beschreibung).
Es wurde gefunden, dass sich diese Klassen u.a. aus den Blutungsdaten der Beobachtungszeitspanne (2), die mit dem Einsetzen der Hormonspirale beginnt und bis Tag 90 dauert, vorhersagen lassen. Zur Vorhersage wurde ein Klassifizierungsmodell entwickelt, das auf Basis von Prädiktoren vorhersagt, in welche Blutungsverhaltensklasse eine Frau fällt. Das Klassifizierungsmodell wurde mit Hilfe einer Random-Forest-Methode entwickelt.
Die Random-Forest Methode ist ein Verfahren des Maschinenlernens, welches beispielsweise als Klassifikations- oder Regressionsverfahren angewandt werden kann. Es basiert auf der Konstruktion mehrerer Entscheidungsbäume. Diese zählen ebenfalls zu den Klassifikationsverfahren.
Das Ziel eines Entscheidungsbaums ist es, ein Model zu erstellen, welches die Klasse oder Kategorie einer Zielvariable automatisiert basierend auf Prädiktorvariablen vorhersagt. Ein Entscheidungsbaum besteht immer aus einem Wurzelknoten und beliebig vielen inneren Knoten sowie mindestens zwei Blättern. Dabei repräsentiert jeder Knoten eine logische Regel und jedes Blatt eine Antwort auf das Entscheidungsproblem. Steht nun eine Vielzahl von Prädiktorvariablen zur Verfügung, wird für den ersten Knoten des Baumes diejenige Variable gewählt, welche zur "besten" Teilung der Population bezüglich der Zielvariablen führt. Dies kann beispielsweise durch Kriterien wie dem Gini-Koeffizient oder Information Gain kalkuliert werden. Der Baum "wächst" dann so lange weiter, bis sich kein weiterer Informationsgewinn durch das Hinzufügen weiterer Variablen erzielen lässt.
Der Random Forest Algorithmus ist eine Abwandlung des einfachen Entscheidungsbaums. Anstelle eines einfachen Baums, werden viele (z.B. 1000, 10000) unterschiedliche Entscheidungsmodelle berechnet. Dazu wird zunächst für jeden Baum zufällig eine bestimmte Anzahl an Variablen aus der Gesamtheit der Prädiktorvariablen gewählt. Basierend auf dieser Menge wird dann ein Entscheidungsbaum berechnet. Dieses Vorgehen wird im Random Forest Algorithmus mehrfach wiederholt, sodass am Ende des Verfahren ein "Wald" steht. Soll nun eine neue Beobachtung anhand des Models klassifiziert werden, wird die Vorhersage von jedem einzelnen Baum berechnet und dann geprüft, welche Klasse von den meisten Bäumen vorhergesagt wurde. Diese geht dann als Vorhersageergebnis aus dem Modell hervor.
Da es im vorliegenden Fall einen Unterschied der Auswirkungen von Fehlklassifikationen gibt, wurde außerdem eine Kostenmatrix zur Erstellung des Models integriert. Dabei wurden Fehlklassifikationen, welche "weiter weg" von der wahren Klasse sind doppelt so schwer gewichtet wie Fehlklassifikationen, welche in die benachbarten Klassen fallen.
Möchte nun eine "neue" Probandin, welche nicht in den Modelberechnungen enthalten ist, Informationen über ihr zukünftiges Blutungsmuster erhalten, werden die Ausprägungen der Prädiktorvariablen sowie das Blutungsverhalten der ersten 90 Tage verwendet. Die Probandin erhält dann eine Information darüber, welche Blutungsklasse die größte Wahrscheinlichkeit aufweist einzutreten.
Das berechnete Model wurde basierend auf 73 Prädiktorvariablen und 1351 Beobachtungen berechnet. Zur Berechnung der Fehlerraten und Wahrheitsmatrizen wurde eine drei-fache Kreuzvalidierung durchgeführt. Zur weiteren Sensitivitätsüberprüfung des finalen Models wurde dieses auf zwei weiteren Datensätzen getestet. Diese Daten stammen aus klinischen Studien zu weiteren intrauterines Wirkstofffreisetzungssystemen.
Die Genauigkeit der Vorhersage wurde anhand einer 1000-fachen Durchführung einer dreifachen Kreuzvalidierung berechnet. Dabei ergab sich eine durchschnittliche Rate korrekter Klassifikationen von 70,4%. Die Wahrheitsmatrix ist im Folgenden abgebildet (Tabelle 2). Hierbei ist darauf hinzuweisen, dass das Modell so angepasst wurde, dass auftretende Fehlklassifikationen möglichst in "benachbarte" Cluster fallen.

**Tabelle 2: Wahrheitsmatrix**

| | | **Vorhergesagte Cluster** | | | |
|---|---|---|---|---|---|
| | | Vorwiegendes Blutungsmuster: Amenorrhoe | Vorwiegendes Blutungsmuster: Schmierblutungen | Vorwiegendes Blutungsmuster: Blutungen | Total |
| **Wahre Cluster** | Vorwiegendes Blutungsmuster: Amenorrhoe | 55 | 71 | 12 | 139 |
| | Vorwiegendes Blutungsmuster: Schmierblutungen | 22 | 324 | 132 | 478 |
| | Vorwiegendes Blutungsmuster: Blutungen | 5 | 157 | 572 | 734 |
| | Total | 82 | 552 | 717 | 1351 |
| % richtige Vorhersage* | | 67,1% | 58,7% | 79,8% | 70,4% |
| % richtig oder besser als vorhergesagt** | | 67,1% | 71,5% | 100% | 86,4% |

Das finale Model wurde außerdem auf zwei weiteren unabhängigen Datensätzen getestet, welche zuvor nicht in die Modelbildung eingingen. Hier ergaben sich mit 69% und 72,2% ähnlich gute Raten korrekter Klassifikationen.
Tabelle 3 ist eine Zusammenstellung der Prädiktoren, die in die Entwicklung des Klassifizierungsmodells eingeflossen sind:

**Tabelle 3: Prädiktoren zur Vorhersage des Blutungsverhaltens**

| **Art** | **Beschreibung** | **Zeitraum** |
|---|---|---|
| Baselinedaten | Alter | |
| | Rasse | |
| | Land | |
| | Gewicht | |
| | Größe | |
| | BMI | |
| | Alkoholverzehr | |
| | Rauchstatus | |
| | Rauchdosis | |
| | Parität | |
| | Durchschnittliche Dauer der Abbruchblutung | |
| | Durchschnittliche Zykluslänge | |
| | Anzahl Abtreibungen | |
| | Anzahl Geburten | |
| | Anzahl Kaiserschnitte | |
| | Anzahl ektopischer Schwangerschaften | |
| | Anzahl Schwangerschaften | |
| | Anzahl Vaginalgeburten | |
| | Durchschnittliche Intensität der Abbruchblutung | |
| | Zyklusregularität | |
| | Vorherige Verhütungsmethode | |
| | Bildungsniveau | |
| Aus Blutungstagebuch abgeleitete Variablen | Anzahl der Blutungstage | Tag 1-30 |
| | | Tag 31-60 |
| | | Tag 61-90 |
| | | Tag 31-90 |
| | | Tag 1-90 |
| | Anzahl der Blutungs-Schmierblutungstage | Tag 1-30 |
| | | Tag 31-60 |
| | | Tag 61-90 |
| | | Tag 31-90 |
| | | Tag 1-90 |
| | Anzahl der Schmierblutungstage | Tag 1-30 |
| | | Tag 31-60 |
| | | Tag 61-90 |
| | | Tag 31-90 |
| | | Tag 1-90 |
| | Anzahl der Blutungsepisoden | Tag 1-30 |
| | | Tag 31-60 |
| | | Tag 61-90 |
| | | Tag 31-90 |
| | | Tag 1-90 |
| | Anzahl der Schmierblutungsepisoden | Tag 1-30 |
| | | Tag 31-60 |
| | | Tag 61-90 |
| | | Tag 31-90 |
| | | Tag 1-90 |
| | Anzahl der Blutungs- und Schmierblutungsepisoden | Tag 1-30 |
| | | Tag 31-60 |
| | | Tag 61-90 |
| | | Tag 31-90 |
| | | Tag 1-90 |
| | Durchschnittliche Dauer der Blutungs- und Schmierblutungsepisoden | Tag 1-30 |
| | | Tag 31-60 |
| | | Tag 61-90 |
| | | Tag 31-90 |
| | | Tag 1-90 |
| | Durchschnittliche Dauer der Schmierblutungsepisoden | Tag 1-30 |
| | | Tag 31-60 |
| | | Tag 61-90 |
| | | Tag 31-90 |
| | | Tag 1-90 |
| | Maximale Dauer der Blutungs- und Schmierblutungsepisoden | Tag 1-30 |
| | | Tag 31-60 |
| | | Tag 61-90 |
| | | Tag 31-90 |
| | | Tag 1-90 |
| | Maximale Dauer der Schmierblutungsepisoden | Tag 1-30 |
| | | Tag 31-60 |
| | | Tag 61-90 |
| | | Tag 31-90 |
| | | Tag 1-90 |
| | Maximum der Autokorrelationsfunktion | Tag 31-90 |
| | | Tag 1-90 |

Zusätzlich zur Blutungsintensität (Klassen (20), (30), (40)) sollte die Regularität des menstrualen Zyklus einer Frau klassifiziert werden (Klassen (31), (32), (41), (42)). Dazu wurde für die Blutungsintensitäten jeder Frau als Funktion der Zeit die Autokorrelationsfunktion für verschiedene Zeitabstände zwischen 21 und 35 Tagen berechnet. Das Maximum der Autokorrelationsfunktion ergibt die wahrscheinlichste Zykluslänge der jeweiligen Frau. Basierend auf der so ermittelten Zykluslänge werden dann zwei Zeitreihenmodelle je Probandin berechnet. Eins der Modelle beinhaltet eine Saisonkomponente, welche auf der berechneten Zykluslänge basiert. Das andere Model beinhaltet diese Komponente nicht. Anhand der AIC Kriteriums wird überprüft, welches Modell den größeren Informationsgehalt bietet. Ist dies das Model inklusive einer Saisonkomponente, wird der Zyklus der Probandin als regelmäßig definiert. Zeigt das Model ohne Saisonkomponente den besseren Wert, wird der Zyklus als unregelmäßig definiert.
Basierend auf der Berechnung der Zyklusregularität für die Probandinnen des Analysedatensatzes, wurde ein logistisches Lasso-Regressions-Modell angepasst. Dabei wird die Regularität als binäre Zielgröße definiert und alle Prädiktorvariablen als potentielle Einflussgrößen aufgenommen. Mit Hilfe des Lasso-Verfahrens werden die Prädiktoren bezüglich ihres Einflusses auf die Zielgröße selektiert. Das finale logistische Regressionsmodel beinhaltet dann nur die Prädiktoren, die nach der Variablenselektion verbleiben.
Es sind zwei Möglichkeiten bezüglich der Anwendung dieses Modells denkbar. Für die erste Möglichkeit wurden zwei verschiedene Modelle dieser Art berechnet. Das erste Modell wird basierend auf den Daten der Probanden aufgestellt, welchen das Random Forest Modell das Blutungscluster vorhersagt. Das zweite Model wird auf den Probandinnen berechnet, welchen das Schmierblutungscluster vorhergesagt. Dementsprechend werden die Modelle auch in der Vorhersage angewandt. Für eine neue Probandin, welche dem Blutungscluster zugeordnet wird, wird anhand des logistischen Regressionsmodells eine Wahrscheinlichkeit für einen regulären Zyklus berechnet. Für eine Probandin, für die hauptsächlich Schmierblutungen vorhergesagt wird, wird eine solche Wahrscheinlichkeit ebenfalls berechnet, jedoch basierend auf dem logistischen Regressionsmodel, welches auf der Teilpopulation des Schmierblutungsclusters basiert.
Für die zweite Möglichkeit der Anwendung eines logistischen Lasso-Regressionsmodells wurde ein Modell auf den gemeinsamen Daten der Probandinnen angepasst, für die das Random Forest Modell Blutungen oder Schmierblutungen klassifiziert hat. Das so berechnete Modell kann dann genutzt werden, um für eine neu klassifizierte Patientin, welcher das Blutung- oder das Schmierblutungscluster prognostiziert wird, die Wahrscheinlichkeit einer regulären Blutung zu berechnen.

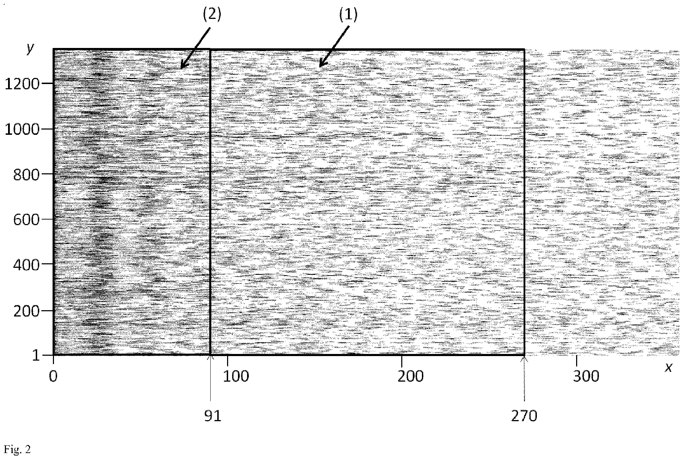

**Fig. 3** zeigt ein Beispiel einer Klassenbildung für die Blutungsdaten in der Vorhersagezeitspanne (1) aus Fig. 2.
Die Klasse (10) umfasst alle 1351 Frauen der Studie. Es erfolgt eine Unterteilung in die Klassen (20), (30) und (40). Klassen (20) umfasst diejenigen Frauen, die weniger als 5% Schmierblutungstage und weniger als 1% Blutungstage in der Vorhersagezeitspanne aufweisen. Frauen, die in dieser Klasse liegen, zeigen überwiegend Amenorrhoe. Der Klasse (20) sind 139 Frauen zugeordnet. Die Klasse (30) umfasst diejenigen Frauen, die weniger als 5% Blutungstage in der Vorhersagezeitspanne aufweisen, aber nicht der Klasse (20) zugeordnet sind. Frauen, die in dieser Klasse liegen, zeigen bei den auftretenden Blutungen vorwiegend Schmierblutungen. Der Klasse (30) sind 478 Frauen zugeordnet. Die Klasse (40) umfasst diejenigen Frauen, die weder Klasse (20) noch Klasse (30) zugeordnet wurden. Frauen, die in dieser Klasse liegen, zeigen bei den auftretenden Blutungen vorwiegend "echte" Blutungen. Der Klasse (40) sind 734 Frauen zugeordnet.
Die Klassen (30) und (40) wurden im Hinblick auf die Regelmäßigkeit der (Schmier-)Blutungen noch weiter unterteilt. Die Klasse (31) umfasst diejenigen Frauen, die regelmäßige Schmierblutungen aufweisen. Die Klasse (32) umfasst diejenigen Frauen, die unregelmäßige Schmierblutungen aufweisen. Die Klasse (41) umfasst diejenigen Frauen, die regelmäßige Blutungen aufweisen. Die Klasse (42) umfasst diejenigen Frauen, die unregelmäßige Blutungen aufweisen.
**Fig. 4** zeigt ein weiteres Beispiel einer Klassenbildung für die Blutungsdaten in der Vorhersagezeitspanne (1) aus Fig. 2.

Dieser Klassenbildung liegt ein anderer Ansatz zugrunde als der Klassenbildung gemäß Fig. 3; der Klassenbildung liegt hier ein Regressionsbaum zugrunde. Das allgemeine Vorgehen ist hier, eine Population bezüglich einer bestimmten Zielvariablen in möglichst unterschiedliche Gruppen zu teilen. Zur Teilung wird eine Variable, die Teil der Prädiktoren ist ausgewählt. Hierbei wird diejenige Variable gewählt, welche den größten Unterschied zwischen den Gruppen bezüglich der Zielvariablen bewirkt. Der Unterschied der beiden Gruppen wird basierend auf einem Maß der Knotenreinheit berechnet und maximiert; der Residuenquadratsumme. Dazu wird ein F-Test Kriterium verwendet, dessen Teststatistik der Quotient der Residuenquadratsummen multipliziert mit 1/(n-1) ist. Dieses Verfahren wird dann mehrfach angewandt, um die Population in mehrere Gruppen bezüglich unterschiedlicher Zielvariablen der Vorhersagezeitspanne zu teilen. Von Interesse sind hier die Anzahl der Blutungstage, die Anzahl der Schmierblutungstage und die Regularität, welche über den maximalen Wert der Autokorrelationsfunktion dargestellt wird.

In einem ersten Schritt wurden innerhalb der ersten 90 Tage nach Einsetzen der Hormonspirale nach Prädiktoren gesucht, welche die Gruppe der 1351 Frauen (Klassen (10)) in zwei möglichst unterschiedliche Gruppen bezüglich der Anzahl der Blutungstage in der Vorhersagezeitspanne unterteilt (Klassen (100) und (200)). In einem zweiten Schritt wurden die Frauen mit mehr Blutungstagen (Klasse (100) in die Gruppen "irregulär" (Klasse (110)) und "regulär" (Klasse (120)) geteilt; die Gruppe der Frauen mit weniger Blutungstagen (Klasse (200)) wurde bezüglich der Anzahl der Schmierblutungstage in eine mit mehr Schmierblutungen (Klasse (210)) und eine mit weniger Schmierblutungen (Klassen (220)) geteilt. Der Klasse (100) sind 495 Frauen zugeordnet. Der Klasse (200) sind 856 Frauen zugeordnet. Der Klasse (110) sind 243 Frauen zugeordnet. Der Klasse (120) sind 251 Frauen zugeordnet. Der Klasse (210) sind 369 Frauen zugeordnet. Der Klasse (220) sind 487 Frauen zugeordnet.

In Tabelle 4 sind die Bedingungen für die Aufteilung der Gruppen zusammengestellt.

**Tabelle 4: Bedingungen für die Aufteilung der Klassen in Fig. 4.**

| | |
|---|---|
| (a) | weniger als 20 Tage mit Blutungen oder Schmierblutungen in den ersten 90 Tagen nach Einsetzen der Hormonspirale |
| (b) | mehr als 19 Blutungstage in den ersten 90 Tagen nach Einsetzen der Hormonspirale |
| (c) | Wert der Autokorrelationsfunktion größer oder gleich 0,3 |
| (d) | Wert der Autokorrelationsfunktion kleiner als 0,3 |
| (e) | mehr als 4 Schmierblutungstage in den ersten 90 Tagen nach Einsetzen der Hormonspirale |
| (f) | weniger als 5 Schmierblutungstage in den ersten 90 Tagen nach Einsetzen der Hormonspirale |

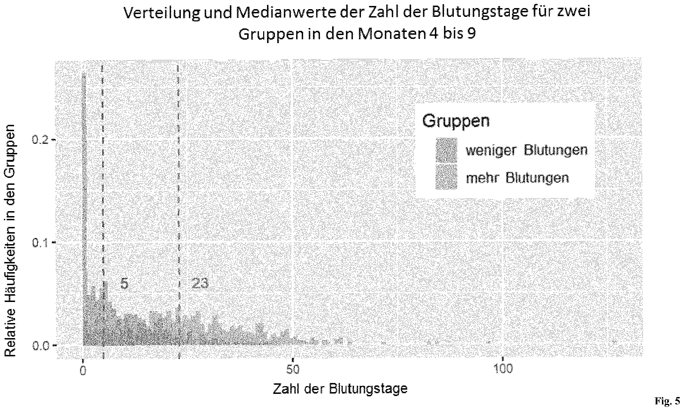

In Fig. 5 sind die Verteilungen der Blutungstage der beiden sich durch diese Regeln ergebenden Gruppen dargestellt. Trotz einer Überlappung ist ein deutlicher Unterschied der beiden Verteilungen erkennbar.

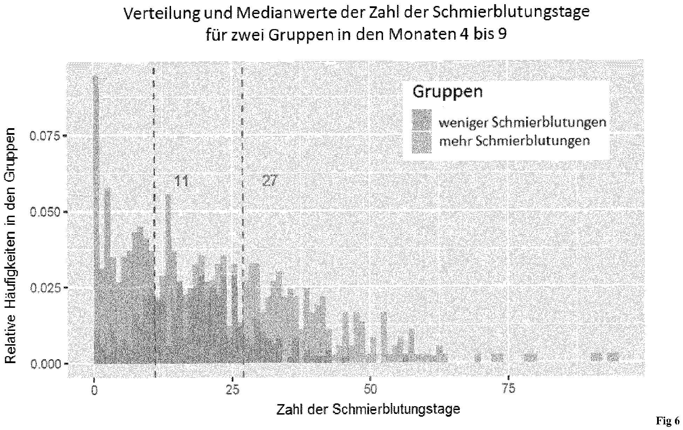

In Fig. 6 sind die Verteilungen der Schmierblutungstage der beiden Gruppen dargestellt, welche sich in der "weniger Blutungstage"-Gruppe ergeben.

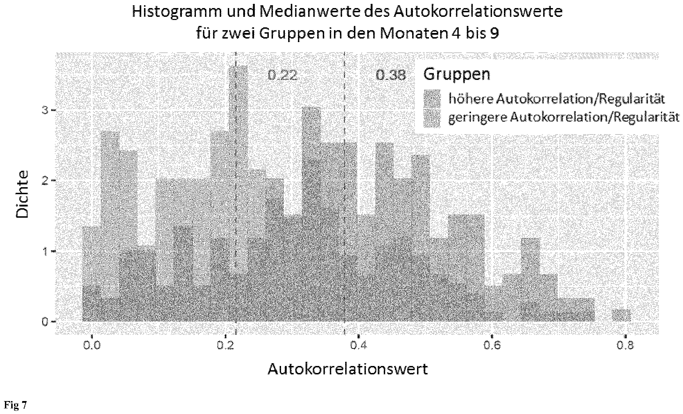

In Fig. 7 sind die Verteilungen der maximalen Werte der Autokorrelationsfunktion der beiden Gruppen dargestellt, welche sich aus der "mehr Blutungstage"-Gruppe ergeben.

Nachfolgend werden bevorzugte Ausführungsformen des erfindungsgemäßen Verfahrens beschrieben.

In einer bevorzugten Ausführungsform wurde einer Frau eine Hormonspirale eingesetzt, die ein Gestagen abgibt. Die Frau lädt sich über einen so genannten App-Store ein erfindungsgemäßes Computerprogrammprodukt als so genannte App auf ihr Smartphone, installiert das Computerprogramm und startet es.

Das Computerprogramm ist so konfiguriert, dass es die Frau begrüßt und sie bittet, einige Angaben zu tätigen, wie beispielsweise ihr Alter und den Zeitpunkt des Einsetzens der Hormonspirale in entsprechende Felder, die eine grafische Benutzeroberfläche bereitstellen, einzutragen oder aus einer Liste auszuwählen oder in einen virtuellen Kalender einzutragen. Die Frau wird nun gebeten, für eine Beobachtungszeitspanne anzugeben, ob sie an den einzelnen Tagen der Beobachtungszeitspanne eine Blutung oder eine Schmierblutung oder keine Blutung hat/hatte. Nach Ablauf der Beobachtungszeitspanne und der Eingabe der Daten übermittelt das Smartphone die erfassten Daten über das Mobilfunknetz und/oder das Internet an ein zweites Computersystem. Auf dem zweiten Computersystem ist ein Vorhersagemodell installiert, das die Prädiktorenwerte in Empfang nimmt und eine Vorhersage über das zukünftige Blutungsverhalten der Frau trifft.

Das Ergebnis der Vorhersage wird von dem zweiten Computersystem an das erste Computersystem übermittelt und der Frau auf dem Display des Smartphones angezeigt.

In einer weiteren bevorzugten Ausführungsform wird die Vorhersage auf Basis neu eingetragener Daten stetig verbessert. Das Computerprogramm ist so konfiguriert, dass es neu eingetragene Daten (Prädiktorenwerte) von Zeit zu Zeit an das zweite Computersystem übermittelt. Das zweite Computersystem aktualisiert die Vorhersage durch Nutzung aller bisher erfassten Prädiktorenwerte und übermittelt das Ergebnis der Vorhersage an das erste Computersystem.

In einer weiteren bevorzugten Ausführungsform ist das Computerprogramm auf dem ersten Computersystem so konfiguriert, dass es die vorhergesagten Tage, an denen eine Blutung auftreten soll, in den virtuellen Kalender einträgt. Die Intensität der vorhergesagten Blutung wird durch eine farbige Kodierung ebenfalls in den Kalender eingetragen. Denkbar ist auch, dass die Unsicherheit über den Eintritt einer Blutung an einem Tag dadurch gekennzeichnet wird, dass in dem Kalender bereits für einen oder mehrere Tage vor und/oder nach dem vorhergesagten Tag die Möglichkeit des Auftretens einer Blutung angezeigt wird.

In einer weiteren bevorzugten Ausführungsform erfolgen Erfassung von Prädiktoren und Berechnung von Vorhersagen auf einem einzigen Computersystem, das durch eine Nutzerin bedient wird, der ein intrauterines Wirkstofffreisetzungssystem eingesetzt worden ist. Die Nutzerin trägt fortwährend Prädiktorwerte in das Computersystem ein. Sie wird dabei durch eine grafische Benutzerschnittstelle und/oder einen Chat-Bot geleitet. Durch Drücken einer virtuellen Taste kann die Nutzerin die Berechnung einer Vorhersage starten. Dabei werden die erfassten Prädiktorenwerte einem Vorhersagemodell, das auf dem Computersystem gespeichert ist, zugeführt. Das Vorhersagemodell errechnet ein zukünftiges Blutungsverhalten und zeigt dieses der Nutzerin an.

In einer weiteren bevorzugten Ausführungsform wird eine Frau, der von einem Arzt ein intrauterines Wirkstofffreisetzungssystem eingesetzt worden ist, von dem Arzt gebeten, während eines Beobachtungszeitraumes ihr Blutungsverhalten zu beobachten und Informationen über das Blutungsverhalten zu erfassen, zum Beispiel mit Hilfe eines Computersystems oder mit Hilfe von Formularen, die die Frau ausfüllen soll. Bei einer Nachuntersuchung überträgt der Arzt die von der Frau gesammelten Informationen über ihr Blutungsverhalten in ein Computersystem und das Computersystem errechnet anhand der Informationen und vorzugsweise anhand von weiteren Informationen über die Frau das zukünftige Blutungsverhalten der Frau. Das Ergebnis wird dem Arzt angezeigt und er kann es mit der Frau diskutieren und ihr das Ergebnis der Vorhersage ausdrucken oder als Datei auf ein Computersystem der Frau übertragen.

## Patentansprüche

1. System zur Vorhersage des Blutungsverhaltens einer Frau, der ein intrauterines Wirkstofffreisetzungssystem eingesetzt worden ist, umfassend
- eine Erfassungseinheit zur Erfassung von Prädiktorenwerten, wobei die Erfassungseinheit so konfiguriert ist, dass sie Informationen zum Blutungsverhalten der Frau in einer Beobachtungszeitspanne erfasst, wobei die Beobachtungszeitspanne mit dem Einsetzen des intrauterinen Wirkstofffreisetzungssystems beginnt und mindestens 60 Tage dauert,
- eine Vorhersageeinheit, die so konfiguriert ist, dass sie das zukünftige Blutungsverhalten der Frau auf Basis der Prädiktorenwerte ermittelt, wobei der Vorhersageeinheit ein Klassifikationsmodell zugrunde liegt, dass die Frau einer von mehreren Blutungsverhaltensklassen zuordnet, wobei sich die Blutungsverhaltensklassen hinsichtlich zu erwartender Blutungsintensitäten in einer Vorhersagezeitspanne unterscheiden, wobei die Vorhersagezeitspanne zeitlich an die Beobachtungszeitspanne anschließt,
- eine Ausgabeeinheit, die so konfiguriert ist, dass sie das ermittelte zukünftige Blutungsverhalten gegenüber der Frau und/oder einer anderen Person, insbesondere gegenüber einem Arzt, anzeigt.

2. System nach Anspruch 1, **dadurch gekennzeichnet, dass** die Erfassungseinheit Bestandteil eines mobilen Computersystems, vorzugsweise eines Smartphones oder Tablet-Computers ist.

3. System nach Anspruch 1 oder 2, umfassend ein erstes Computersystem zur Erfassung der Prädiktorenwerte und ein zweites Computersystem zur Vorhersage des zukünftigen Blutungsverhaltens der Frau, wobei die Computersysteme so konfiguriert sind, dass das erste Computersystem Prädiktoren an das zweite Computersystem übermittelt und das zweite Computersystem ein Ergebnis der Vorhersage an das erste Computersystem übermittelt.

4. System nach Anspruch 1, **dadurch gekennzeichnet, dass** Erfassungseinheit, Vorhersageeinheit und Ausgabeeinheit Bestandteile eines einzigen Computersystems sind.

5. System nach einem der Ansprüche 1 bis 4, ferner umfassend einen oder mehrere Sensoren, die so konfiguriert sind, dass sie einen oder mehrere Signale an die Erfassungseinheit übermitteln, und die Erfassungseinheit so konfiguriert ist, dass sie die Signale als Prädiktorenwerte interpretiert.

6. Verfahren zur Vorhersage des Blutungsverhaltens einer Frau, der ein intrauterines Wirkstofffreisetzungssystem eingesetzt worden ist, umfassend die Schritte
- Erfassen des Blutungsverhaltens der Frau in einer Beobachtungszeitspanne, wobei die Beobachtungszeitspanne mit dem Einsetzen des intrauterinen Wirkstofffreisetzungssystems beginnt und mindestens 60 Tage dauert, und optionales Erfassen weiterer Prädiktorenwerte,
- Ermitteln des zukünftigen Blutungsverhalten der Frau auf Basis der Prädiktorenwerte mit Hilfe eines Klassifikationsmodells, wobei das Klassifikationsmodell die Frau einer von mehreren Blutungsverhaltensklassen zuordnet, wobei sich die Blutungsverhaltensklassen hinsichtlich zu erwartender Blutungsintensitäten in einer Vorhersagezeitspanne unterscheiden, wobei die Vorhersagezeitspanne zeitlich an die Beobachtungszeitspanne anschließt,
- Ausgeben des ermittelten zukünftigen Blutungsverhaltens an die Frau und/oder an eine andere Person, insbesondere an einen Arzt.

7. Verfahren nach Anspruch 6, **dadurch gekennzeichnet, dass** die Beobachtungszeitspanne im Bereich von 61 Tagen bis 120 Tagen, bevorzugt im Bereich von 70 Tagen bis 110 Tagen, noch mehr bevorzugt im Bereich von 80 bis 100 Tagen liegt.

8. Verfahren nach einem der Ansprüche 6 oder 7, **dadurch gekennzeichnet, dass** das Klassifikationsmodell auf einem Random-Forrest-Ansatz basiert und die Frau in eine der drei folgenden Klassen einordnet: Amenorrhoe, Schmierblutungen, Blutungen.

9. Verfahren nach einem der Ansprüche 6 bis 8, **dadurch gekennzeichnet, dass** zusätzlich zu den zu erwartenden Blutungsintensitäten eine Vorhersage zur Regelmäßigkeit der Blutungen und/oder Schmierblutungen erzeugt wird, der eine berechnete Periodenlänge der Frau zugrunde liegt, die auf Basis einer Autokorrelation der Blutungsintensitäten als Funktion der Zeit ermittelt worden ist.

10. Verfahren nach einem der Ansprüche 6 bis 9, **dadurch gekennzeichnet, dass** das Klassifikationsmodell auf einem Regressionsbaum basiert und in die Frau in eine der vier folgenden Klassen einordnet: irreguläre Blutungen, reguläre Blutungen, mehr Schmierblutungen, weniger Schmierblutungen.

11. Verfahren nach einem der Ansprüche 6 bis 10, **dadurch gekennzeichnet, dass** eine erste Vorhersage über das zukünftige Blutungsverhalten auf Basis eines ersten Satzes an Prädiktorenwerten ermittelt wird, die nach Erfassen von weiteren nachfolgenden Pädiktorenwerte ein- oder mehrfach aktualisiert wird.

12. Verfahren nach einem der Ansprüche 6 bis 11, **dadurch gekennzeichnet, dass** Prädiktorenwerte zu einem oder mehreren der nachfolgend aufgeführten Prädiktoren innerhalb des Beobachtungszeitraumes erfasst werden:
- Zeitpunkte, an denen Blutungen aufgetreten sind,
- Zeitspannen, in denen die Blutungen andauerten,
- Intensitäten der Blutungen
- Gesamtzahl der Blutungstage
- Gesamtzahl der Spottingtage
- Regularität der Blutung (bzgl. Dauer und Intensität)
- Anzahl von Blutungs- oder Spottingepisoden.

13. Verfahren nach einem der Ansprüche 6 bis 12, **dadurch gekennzeichnet, dass** Prädiktorenwerte zu einem oder mehreren der nachfolgend aufgeführten Prädiktoren erfasst werden:
- das Alter der Frau,
- die Verhütungsmethode, die die Frau vor dem Einsatz des intrauterinen Wirkstofffreisetzungssystems verwendet hat,
- die Zahl der bisherigen Schwangerschaften/Geburten der Frau,
- die Einnahme von Tabletten und/oder Suchtmitteln,
- Angaben zum Blutungsverhalten in einem definierten Zeitraum vor Einsetzen eines intrauterinen Wirkstofffreisetzungssystems.

14. Verfahren nach einem der Ansprüche 6 bis 13, **dadurch gekennzeichnet, dass** das zukünftige Blutungsverhalten in einen virtuellen Kalender eingetragen wird.

15. Computerprogrammprodukt umfassend einen Datenträger, auf dem ein Computerprogramm gespeichert ist, das in den Arbeitsspeicher eines oder mehrerer Computersysteme geladen werden kann und dort das Computersystem/die Computersysteme dazu veranlasst, folgende Schritte ausführen:
- Erfassen des Blutungsverhaltens einer Frau, der ein intrauterines Wirkstofffreisetzungssystem eingesetzt worden ist, in einer Beobachtungszeitspanne, wobei die Beobachtungszeitspanne mit dem Einsetzen des intrauterinen Wirkstofffreisetzungssystems beginnt und mindestens 60 Tage dauert, und optionales Erfassen weiterer Prädiktorenwerte,
- Ermitteln des zukünftigen Blutungsverhalten der Frau auf Basis der Prädiktorenwerte mit Hilfe eines Klassifikationsmodells, wobei das Klassifikationsmodell die Frau einer von mehreren Blutungsverhaltensklassen zuordnet, wobei sich die Blutungsverhaltensklassen hinsichtlich zu erwartender Blutungsintensitäten in einer Vorhersagezeitspanne unterscheiden, wobei die Vorhersagezeitspanne zeitlich an die Beobachtungszeitspanne anschließt,
- Ausgeben des ermittelten zukünftigen Blutungsverhaltens an die Frau und/oder an eine andere Person, insbesondere an einen Arzt.
